# EUROPEAN PATENT SPECIFICATION

(11) **EP 4 595 995 B1**
(45) Date of publication and mention of the grant of the patent: **04.11.2026**
(21) Application number: 24154981.5
(22) Date of filing: 31.01.2024
(51) Int. Cl.: A61M 5/00, B65G 17/46

(54) **HOLDING CONTAINERS FOR OVEN BAKING OF SYRINGES WITH SILICONE COATING**
HALTEBEHÄLTER FÜR DAS OFENBACKEN VON SPRITZEN MIT SILIKONBESCHICHTUNG
CONTENANTS DE MAINTIEN POUR LA CUISSON AU FOUR DE SERINGUES AVEC REVÊTEMENT DE SILICONE

(43) Date of publication of application: 06.08.2025
(73) Proprietor: Becton, Dickinson and Company, Franklin Lakes, NJ 07417-1880 (US)
(72) Inventor: RENZETTI, Philippe, 38920 Crolles (FR)
(74) Representative: Germain Maureau

(56) References cited:
- EP-A1- 2 110 345
- EP-A1- 3 597 573
- DE-A1- 10 318 731

## Description

### BACKGROUND OF THE INVENTION

### Field of the Invention

The present disclosure relates generally to syringes and, more particularly, to syringe holding pucks employed for the manufacture of syringe barrels having a baked silicone coating thereon, with the holding pucks supporting the syringe barrels to prevent or minimize weakening of the syringe barrel tip that can occur during baking thereof.

### Description of Related Art

Document EP 3 597 573 A1 discloses a syringe barrel and holding puck arrangement. Syringes are used in a variety of environments for administering fluids, such as medications or other drugs, to a patient. A syringe will typically include a glass (or plastic) syringe barrel with an opening at a distal end and a plunger assembly inserted through the opposite proximal end of the barrel. The distal end of the syringe barrel may include a tip through which the opening is formed. In some embodiments, the tip may be configured as a luer connection that enables a transfer of fluid out from or into the syringe barrel, while in other embodiments the tip may be configured as a hub that retains a needle therein for the syringe. The plunger assembly typically includes an elongated plunger rod extending out of the barrel and a plunger head or stopper disposed at the distal end of the plunger rod. The stopper is typically made of elastomeric material and is adapted to ensure the container closure integrity of a syringe when the stopper is inserted into the syringe. The plunger assembly is movable within the syringe barrel to force fluid out from an inner volume of the syringe barrel and/or to aspirate fluid into the inner volume of the syringe barrel - with the stopper sliding through the barrel when the plunger assembly is actuated by a user.

In order to improve sliding of the stopper within the barrel, it is known in the art to lubricate the barrel with a lubricant coating, such as a silicone coating as a primary example. The coating can be deposited on the inner walls of the barrel by spraying, dipping or another suitable application technique. The siliconization of the inner wall of the syringe barrel allows a smooth movement or gliding of the stopper within the chamber, but it has been found that in some cases the silicone coating may interact with components of the drug or medicament contained within the syringe barrel and alter the drug properties. In order to prevent such interaction of the silicone coating with the drug/medicament, it is common to treat the silicone coating applied to the syringe barrel. As an example, for a silicone coating that comprises an emulsion of poly-(dimethylsiloxane), it is common for the coating to be annealed to form so-called "baked silicone", in order to make the coating less reactant with the drug/medicament contained in the syringe barrel.

In the manufacture of syringe barrels that include a baked silicone coating thereon, a large number of silicone-lubricated syringe barrels are typically loaded into an arrangement of holders or "pucks" that provide for the transfer of the syringe barrels into an oven that heat-treats the syringe barrels to anneal the silicone coating. The holding pucks may be provided in an array of rows and columns into which syringes barrels may be loaded, with the holding pucks arranged on a conveyor system that functions to move the holding pucks into and through an oven for heat treatment of the syringe barrels.

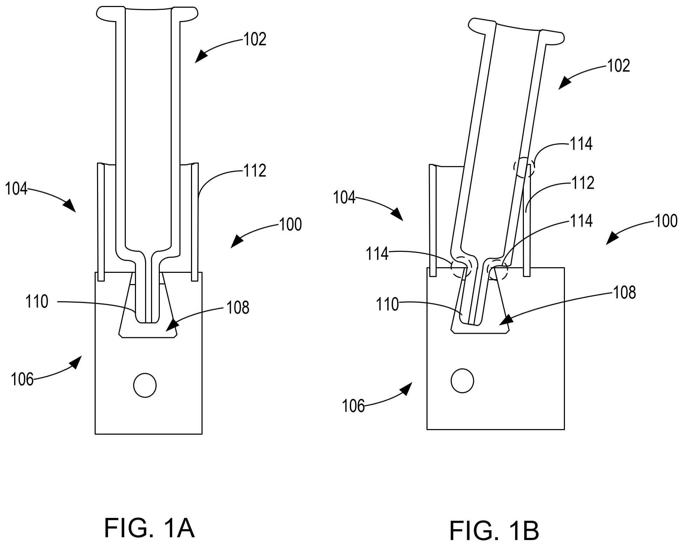

A known syringe holding puck 100 is shown in FIGS. 1A and 1B according to the prior art. The holding puck 100 is formed of a metallic material that is durable at high temperature, so as to be suitable for retaining a syringe barrel 102 during oven baking of the syringe barrel 102 to anneal the silicone coating. The holding puck 100 may be generally characterized as including an upper barrel holder 104 and a lower tip holder 106. The lower tip holder 106 is formed of a generally solid base that includes a chamber 108 therein that is sized and configured to receive a tip 110 of the syringe barrel 102 therein. In some embodiments, the chamber 108 has a frustoconical shape that tapers/slopes from a bottom end thereof to a top end thereof, with the top end of the frustoconical chamber 108 opening into the upper barrel holder 104. The upper barrel holder 104 is formed of a cylindrical sidewall 112 that is positioned on top of the base and extends upwardly therefrom. The upper barrel holder 104 has an open top end to provide for insertion of the syringe barrel 102 into the holding puck 100, along with an open bottom end that provides access into the chamber 108 of lower tip holder 106.

As shown in FIGS. 1A and 1B, a syringe barrel 102 is initially inserted into the holding puck 100 through the open top end of the upper barrel holder 104 (FIG. 1A), with the syringe barrel 102 then being placed into and supported by the holding puck 100 (FIG. 1B). The syringe barrel 102 is thus retained by holding puck 100, with the tip 110 of the syringe barrel 102 positioned within the chamber 108 of lower tip holder 106 and a main portion of the syringe barrel 102 positioned within a volume defined by the upper barrel holder 104. With the syringe barrel 102 being supported and retained by holding puck 100, the syringe barrel 102 is held at an angle - with the tip 110 tilted toward one of the angled sidewalls of the frustoconical chamber 108 and a main portion of the syringe barrel 102 resting against a top edge of the cylindrical sidewall 112 of upper barrel holder 104. When in this orientation, it is recognized that a number of pressure points (indicated at 114 in FIG. 1B) are created on the syringe barrel 102 where the barrel 102 comes into contact with the holding puck 100 - and that the glass-on-metal contact at these pressure points114 can negatively impact the structural integrity of the syringe barrel 102. In particular, the glass-on-metal contact at "tip grooves" on either side of the syringe tip 110 (wherein the main cylindrical body of the syringe barrel 102 narrows and transitions to the tip 110) may negatively impact the tip strength/resistance of the syringe barrel 102 (i.e., weakens the tip) - making the tip 110 more susceptible to breaking.

Accordingly, a need exists in the art for syringe barrel holding pucks that are able to retain the syringe barrel in a desired orientation, while avoiding or minimizing glass-on-metal contact between the syringe barrel and the holding puck at critical areas (such as the tip grooves) of the syringe barrel. The syringe holding pucks retain and support the syringe barrels therein while a baked silicone coating is annealed on the syringe barrel via an oven-baking process.

### SUMMARY OF THE INVENTION

Provided herein is a syringe barrel and a holding puck wherein the syringe barrel has a cylindrical main body and a tip positioned at a distal end of the cylindrical main body and the holding puck is configured for retaining the syringe barrel. The holding puck includes a base member and a barrel holder mounted on the base member that defines a receptacle for receiving at least part of the syringe barrel. The barrel holder includes a cylindrical sidewall having a top end and a bottom end and, with the cylindrical sidewall oriented along a longitudinal axis extending between the top end and a bottom end, and with the top end defining a top opening. The barrel holder also includes a bottom wall positioned at the bottom end of the cylindrical sidewall, the bottom wall including a bottom opening formed therein, with the bottom opening having a diameter that is less than a diameter of the top opening. The holding puck further includes a support bracket affixed to the base member and including a support platform positioned beneath the barrel holder and oriented orthogonal to the longitudinal axis, with the support platform aligned with the bottom opening. With a syringe barrel retained in the holding puck, a multi-point contact is made between the syringe barrel and the holding puck, with the multi-point contact including a first contact point between a distal end of the tip and the support platform and a second contact point between a side surface of the tip and an inner edge of the bottom wall that defines the bottom opening.

In certain configurations, the multi-point contact further includes a third contact point between a side surface of the cylindrical main body of the syringe barrel and the top end of the cylindrical sidewall of the barrel holder.

In certain configurations, the base member comprises a base plate oriented parallel to the longitudinal axis, the base plate including a first extension member and a second extension member spaced apart to define U-shaped opening.

In certain configurations, the cylindrical sidewall includes a pair of notches formed on an outer surface thereof, on opposing sides of the cylindrical sidewall, and wherein the first extension member and the second extension member interfit with the pair of notches to secure the barrel holder to the base plate.

In certain configurations, the support platform is positioned within the U-shaped opening defined by the first extension member and the second extension member.

In certain configurations, the support bracket comprises a connecting arm extending downward from the support platform, with the connecting arm secured to the base plate.

In certain configurations, the bottom wall of the barrel holder is sloped downwardly and inwardly from the cylindrical sidewall to the bottom opening.

In certain configurations, a diameter of the bottom opening is greater than a diameter of the tip and less than a diameter of the cylindrical main body of the syringe barrel.

In certain configurations, the support platform supports a majority of a weight of the syringe barrel at the first contact point between the distal end of the tip and the support platform.

In certain configurations, the support bracket is formed of stainless steel.

In certain configurations, the tip of the syringe barrel comprises a luer connection or luer fitting.

Also provided herein is a system for performing a silicone lubricant annealing of a plurality of syringe barrels according to claim 12.

In certain configurations, the base member comprises a singular base plate extending along a length of a row of holding pucks of the array of holding pucks, with each of the barrel holders in the row of holding pucks secured to the singular base plate.

In certain configurations, the support bracket comprises a singular support bracket extending along the length of the row of holding pucks, with the singular support bracket affixed to the singular base plate, and wherein the singular support bracket includes support platforms thereon corresponding to each of the holding pucks in the row of holding pucks

In certain configurations, the conveyor comprises a belt and a plurality of mounting brackets arranged along a length of the belt, and wherein a respective pair of mounting brackets of the plurality of mounting brackets are attached on opposing sides of the belt and are affixed to the singular base plate, so as to couple the respective row of holding pucks to the conveyor and provide for advancement thereof.

### BRIEF DESCRIPTION OF THE DRAWINGS

FIGS. 1A and 1B are side cross-sectional views of a syringe barrel being retained by a holding puck, as known in the prior art.
FIG. 2 is a perspective view of a syringe barrel with which embodiments of the disclosure may be implemented;
FIG. 3 is a block schematic diagram of a system for performing a silicone lubricant annealing of a plurality of syringe barrels, according to a non-limiting embodiment described herein;
FIG. 4 is a perspective view of a portion of a retention and transport system included in the system of FIG. 3, according to a non-limiting embodiment described herein;
FIG. 5 is a perspective view of a portion of a retention and transport system included in the system of FIG. 3, according to a non-limiting embodiment described herein;
FIG. 6 is a side cross-sectional view of a holding puck taken along line 6-6 of FIG. 5, according to a non-limiting embodiment described herein; and
FIG. 7 illustrates the holding puck of FIG. 6, with force vectors provided indicated forces applied to the syringe barrel when retained thereby.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art within the scope of the appended claims.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

Aspects and embodiments of the disclosure are directed to syringe barrel holding pucks that may be employed to retain syringe barrels therein as part of an annealing process for providing/forming a baked silicone lubricant coating on the syringe barrels. The holding pucks provide for retention and transport of the syringe barrels into an oven for performing of the baked silicone annealing, while eliminating/minimizing the glass-on-metal contact between the syringe barrel and the holding puck at critical areas (e.g., at tip grooves) of the syringe barrel so as to maintain a tip strength/resistance of the syringe barrel and reduce the potential for tip breakage.

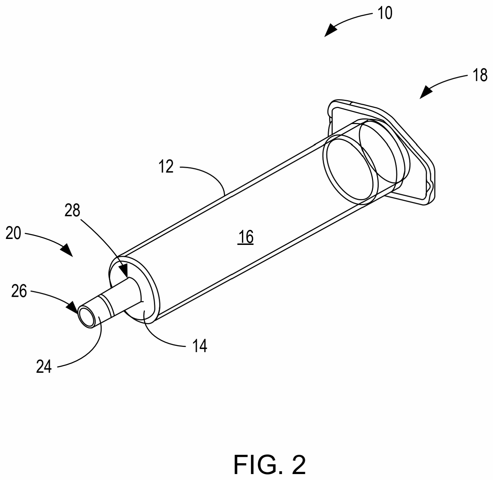

Referring to FIG. 2, shown is a non-limiting embodiment of a syringe barrel 10 with which aspects or embodiments of the disclosure may be implemented. The syringe barrel 10 may be part of a syringe that also includes a plunger assembly (not shown), with the plunger assembly being axially movable within the syringe barrel 10 to an advanced position to facilitate administering of an injectable fluid (e.g., medication) to a patient, for example. As known in the art, the plunger assembly may include a plunger rod having a stopper attached to a distal end thereof, with stopper formed from a material that is different from the material of the plunger rod, such as rubber or another elastomeric material, and that is capable of forming a tight seal with the syringe barrel 10 as it is advanced therethrough.

The syringe barrel 10 is formed of a cylindrical main body 12 and an end wall 14 that collectively define a chamber 16 for retaining fluid therein. The syringe barrel 10 includes an open proximal end 18 configured to receive a plunger assembly therein and a distal end 20 at which end wall 14 is positioned. The proximal end 18 of the syringe barrel 10 may include a flange 22 to facilitate handling and positioning of the syringe 10 and to maintain the relative position of the syringe barrel 10 with respect to the plunger assembly during medication administration. In one embodiment, the distal end 20 of syringe barrel 10 may include a tip 24 that extends distally outward from the end wall 14 and defines an opening or lumen 26 in fluid communication with the chamber 16. In some embodiments, and as illustrated in FIG. 2, the tip 24 is configured as a luer connection or luer fitting that may mate with a corresponding luer connection or luer fitting of a vascular access device (when administering fluid from the syringe barrel) or a fluid source (when aspirating fluid into the syringe barrel). It can be appreciated that, in other embodiments, tip 24 may be provided as a hub within which a needle is secured (i.e., a staked needle syringe) and extends out distally therefrom. As shown in FIG. 2, a tip groove or shoulder 28 is formed about tip 24 where the end wall 14 transitions to the tip 24.

As previously described, it is known in the art to lubricate a syringe barrel 10 with a lubricant coating in order to improve sliding of a plunger assembly stopper within the barrel. A silicone coating, such as an emulsion of poly-(dimethylsiloxane) as a primary example, may be deposited on the inner walls of the syringe barrel 10 to allow a smooth movement or gliding of the stopper within the chamber 16. In order to prevent an interaction of the silicone coating with the drug/medicament contained within the syringe barrel 10, it is common to for the coating to be annealed to form so-called "baked silicone", in order to make the coating less reactant with the drug/medicament contained in the syringe barrel 10.

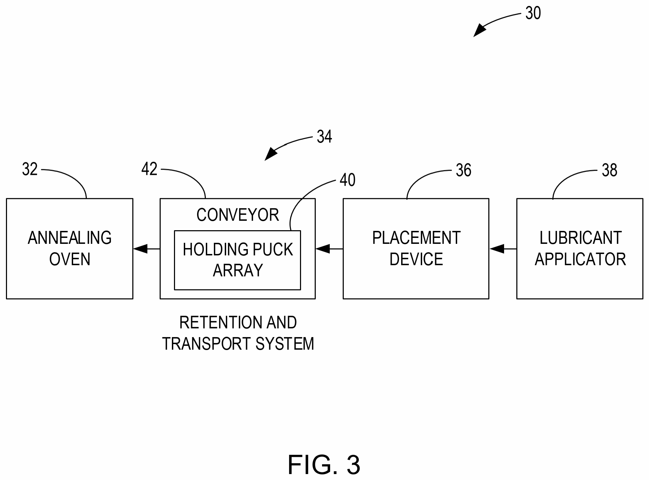

Referring now to FIG. 3, a block schematic diagram of a system 30 for performing a silicone lubricant annealing on/for syringe barrels is illustrated according to an embodiment of the disclosure. The system 30 may be characterized as generally including an oven 32, a retention and transport system 34 for retaining the syringe barrels 10 and advancing the syringe barrels 10 into the oven 32, a placement device 36 for providing syringe barrels 10 to the retention and transport system 34, and a lubricant applicator 38 for applying a silicone lubricant to an inner surface of the syringe barrels 10.

In operation of the system 30, the lubricant applicator 38 may initially operate to apply a silicone lubricant coating (e.g., poly-(dimethylsiloxane)) to the inner surface of the syringe barrels 10. This application of the silicone lubricant to the syringe barrels 10 may be performed while the syringe barrels 10 are held by the placement device 36 or prior to retention of the syringe barrels 10 by the placement device 36. Upon application of the silicone lubricant coating onto the syringe barrels 10, the placement device 36 then delivers the syringe barrels 10 to the retention and transport system 34. In particular, the placement device 36 may deposit a plurality of syringe barrels 10 into an array of holding pucks 40 of the retention and transport system 34 that are configured to receive and retain the syringe barrels 10 therein. Upon receipt of the syringe barrels 10 into the array of holding pucks 40, the holding pucks 40 may be advanced along a conveyor 42 of the retention and transport system 34 - with the conveyor 42 delivering the syringe barrels 10 to the oven 32. The oven 32 functions to bake the silicone coating that has been applied to the syringe barrels 10, in order to anneal the silicone coating and form a baked silicone coating on the inner surface of the syringe barrels 10.

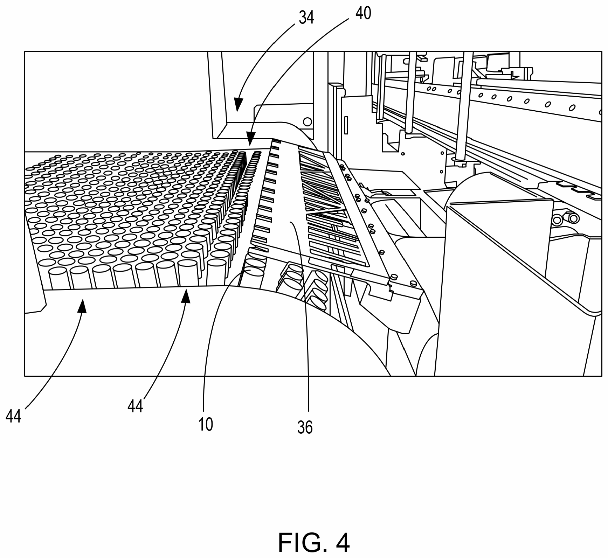

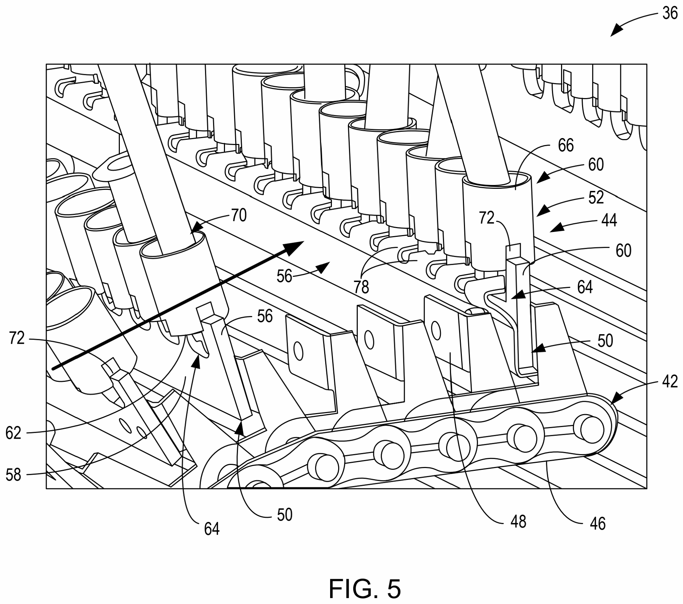

Referring now to FIGS. 4-6, the retention and transport system 34 is shown in greater detail, along with the construction of each individual holding puck 40 of the retention and transport system 34. As shown in FIGS. 4 and 5, according to embodiments of the disclosure, the array of holding pucks 40 may be arranged into a plurality of rows 44 of holding pucks 40 that are affixed to conveyor 42 to provide for movement thereof. In some embodiments, a belt 46 of the conveyor 42 may include a plurality of mounting brackets 48 arranged along a length thereof and on each of opposing sides of the belt 46. Each of a pair of mounting brackets 48 on opposing sides of the belt 46 may be used to secure a respective row 44 of holding pucks 40 to the conveyor 42. In particular, a base member 50 (that may be considered part of holding pucks 40) may be provided that runs a length of the row 44 of holding pucks 40, with the base member 50 secured to a respective pair of mounting brackets 48, so as to affix the row 44 of holding pucks 40 to the conveyor 42.

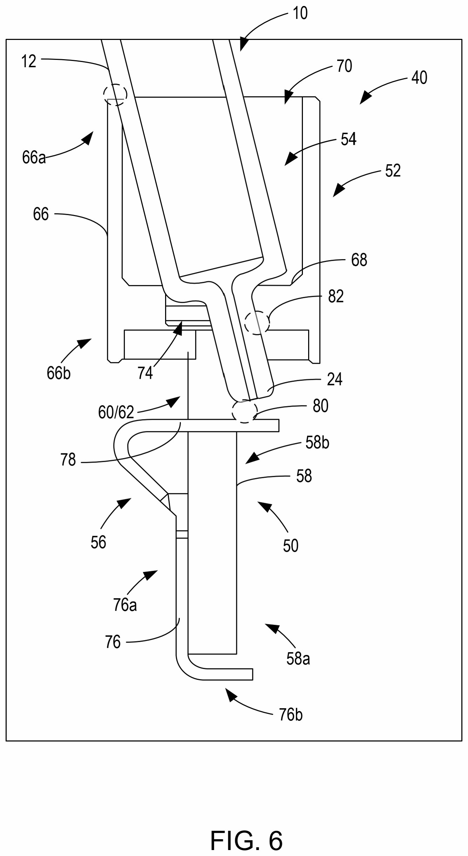

The structure of each of the holding pucks 40 is illustrated in FIGS. 5 and 6, with it shown therein that each of the holding pucks 40 is generally composed of a base member 50, a barrel holder 52 mounted on the base member 50 that defines a receptacle 54 for receiving at least part of the syringe barrel, and a support bracket 56 affixed to the base member 50. One or more of the base member 50, barrel holder 52, and support bracket 56 of each holding puck 40 may be formed of a metallic or composite material that is compatible with the annealing process that is performed on the syringe barrels 10. In a non-limiting example, each of the base member 50, barrel holder 52, and support bracket 56 may be formed of stainless steel. As described in further detail below, the barrel holder 52 and support bracket 56 of the holding puck 40 function collectively to retain and support a syringe barrel 10 therein in a manner that eliminates/minimizes glass-on-metal contact between the syringe barrel 10 and the holding puck 40 at critical areas of the syringe barrel 10, such as at tip grooves 28 on opposing sides of the tip 24 (where tip 24 joins end wall 14), so as to maintain a tip strength/resistance of the syringe barrel 10 and reduce the potential for tip breakage.

According to aspects of the disclosure, the base member 50 of holding puck 40 may be configured as a base plate 58 having a planar construction - with the base plate 58 oriented vertically so as to be in parallel with a longitudinal axis A_{L} of the holding puck 40 and barrel holder 52. The base plate 58 may be considered part of a singular holding puck 40 but, as previously indicated, a common/singular base plate 58 or base member 50 may be shared between all of the holding pucks 40 of a respective row 44 of holding pucks 40 - with the base plate 58 running a length of the row 44 of holding pucks 40 and being affixed at opposing ends thereof to mounting brackets 48 of the conveyor 42. While a bottom portion 58a of the base plate 58 is configured to be affixed to the mounting brackets 48 of the conveyor 42, an upper portion 58b of the base plate 58 is constructed to include a first extension member 60 and a second extension member 62 (for each holding puck 40) that are spaced apart to define U-shaped opening 64, with the extension members 60, 62 extending upward from the bottom portion 58a of the base plate 58.

The barrel holder 52 of each holding puck 40 includes a cylindrical sidewall 66 and a bottom wall 68 that collectively define a receptacle 54 for receiving a portion of the syringe barrel 10 therein. The cylindrical sidewall 66 includes a top end 66a and a bottom end 66b and is oriented along the longitudinal axis A_{L} of the holding puck 40 (the longitudinal axis A_{L} extending between the top end 66a and the bottom end 66b). The top end 66a of the cylindrical sidewall 66 defines a top opening 70 of the barrel holder 52, with the top opening 70 sized to allow for placement of the syringe barrel 10 into the receptacle 54 of the barrel holder 52. In some embodiments, the cylindrical sidewall 66 of barrel holder 52 includes a pair of notches 72 formed on an outer surface thereof to provide for attachment of the barrel holder 52 to the base plate 58. The notches 72 are formed on opposing sides of the cylindrical sidewall 66 (i.e., arranged 180 degrees apart) and are configured to receive the first extension member 60 and the second extension member 62 of base plate 58 therein. The first extension member 60 and the second extension member 62 interfit with the pair of notches 72 to secure the barrel holder 52 to the base plate 58.

The bottom wall 68 of the barrel holder 52 is positioned at the bottom end 66b of the cylindrical sidewall 66. The bottom wall 68 includes a (bottom) opening 74 formed therein that is centered in the bottom wall 68. The bottom opening 74 has a diameter that is less than a diameter of the top opening 70, with the bottom opening 74 sized to enable the tip 24 of the syringe barrel 10 to extend therethrough (i.e., the diameter of the bottom opening 74 is greater than a diameter of the tip 24) but to prevent the cylindrical main body 12 of the syringe barrel 10 from passing therethrough. In some embodiments, the bottom wall 68 may be configured as a sloped bottom wall 68 that is angled downwardly and inwardly from where the bottom wall 68 is joined to the cylindrical sidewall 66 to the bottom opening 74, in order to better accommodate the distal end 20 of the syringe barrel 10, including the tip 24, the end wall 14 of the cylindrical main body 12, and a tip groove 28 formed on each of opposing sides of the tip 24 where it is joined to the end wall 14.

The support bracket 56 of holding puck 40 may be configured as a curved member that generally includes a connecting arm 76 at a lower end thereof and a support platform 78 at an upper end thereof. The connecting arm 76 provides for attachment of the support bracket 56 to the base plate 58, with a first portion 76a of the connecting arm 76 being aligned in parallel with the base plate 58 and configured for attachment to the bottom portion 58a of the base plate 58. In some embodiments, the connecting arm 76 may include a second portion 76b positioned at a bottom end of the first portion 76a, with the second portion 76b arranged orthogonal to the first portion 76a. With the support bracket 56 secured to the base plate 58, the second portion 76b may bend/extend underneath a bottom edge of the base plate 58.

The support platform 78 is positioned above the connecting arm 76 of the support bracket 56, with the connecting arm 76 extending downward from the support platform 78. The support platform 78 is oriented orthogonal to the first portion 76a of connecting arm 76 and to base plate 58 (i.e., orthogonal to the longitudinal axis A_{L}). The support platform 78 is sized and positioned relative to connecting arm 76 such that it extends under the barrel holder 52 (that is also secured to base plate 58), with the support platform 78 being positioned within the U-shaped opening 64 defined by the first extension member 60 and the second extension member 62 of the base plate 58 and being aligned with and positioned underneath the bottom opening 74 of the barrel holder 52.

As shown in FIG. 5, similar to the base plate 58, the support bracket 56 may be considered part of a singular holding puck 40, but a common/singular support bracket 56 (i.e., a common connecting arm 76) may be shared between all of the holding pucks 40 of a respective row 44 of holding pucks 40 - with the connecting arm 76 of support bracket 56 running a length of the row 44 of holding pucks 40 and being affixed to the base plate 58 at a plurality of locations. The common/singular support bracket 56 includes a plurality of support platforms 78 thereon - with each support platform 78 corresponding to a respective holding puck 40 and being aligned relative to the barrel holder 52 thereof as described in detail above.

In accordance with other embodiments of the disclosure, a plurality of individual support brackets 56 may be provided on base plate 58. Each of the support brackets 56 may comprises an inverted U-shaped member having a support platform 78 - with each support bracket 56 being positioned within a respective U-shaped opening 64 defined by the first extension member 60 and the second extension member 62 of the base plate 58.

According to aspects of the disclosure, with a syringe barrel 10 retained in a holding puck 40, a multi-point contact is made between the syringe barrel 10 and the holding puck 40 - with the multi-point contact avoiding glass-on-metal contact between the syringe barrel 10 and the holding puck 40 at what are deemed to be "critical areas" of the syringe barrel 10. As a primary example, glass-on-metal contact between the syringe barrel 10 and the holding puck 40 is prevented at tip grooves or shoulders 28 on opposing sides of the tip 24, where the tip 24 joins the end wall 14 (FIG. 2). Instead, as shown in FIG. 6, the multi-point contact between the syringe barrel 10 and the holding puck 40 may include a first contact point 80 between a distal end of the tip 24 and the support platform 78, a second contact point 82 between a side surface of the tip 24 and an inner edge of the bottom wall 68 that defines the bottom opening 74, and a third contact point 84 between a side surface of the cylindrical main body 12 of the syringe barrel 10 and the top end 66a of the cylindrical sidewall 66 of the barrel holder 52.

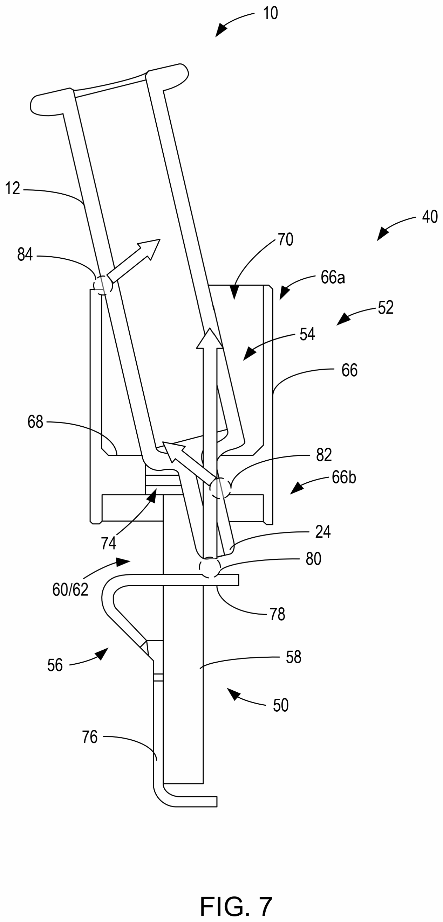

Referring now to FIG. 7, the retaining of a syringe barrel 10 within a holding puck 40 is shown, with force vectors added at the contact points 80, 82, 84 between the syringe barrel 10 and the holding puck 40, so as to illustrate forces applied to the syringe barrel 10 at the contact points.

As shown in FIG. 7, at the first contact point 80 between the distal end of the tip 24 and the support platform 78, a large force is applied to the extremity of the tip 24, thus indicating that the support platform 78 supports a large part of the weight of the syringe barrel 10 at the first contact point 80. The force applied to the tip 24 at the first contact point 80 is oriented along the longitudinal axis A_{L} (i.e., y-axis), such that this force may be absorbed by a length of the tip 24 and there is little to no negative impact on the tip strength/resistance of the syringe barrel 10 by glass-on-metal contact at the first contact point 80.

As also shown in FIG. 7, at the second contact point 82 between the side surface of the tip 24 and the inner edge of the bottom wall 68 that defines the bottom opening 74, a small force is applied to the side of the tip 24. This smaller force applied to the side surface of the tip 24 at the second contact point 82 is angled relative to the longitudinal axis (e.g., at 45 degrees), and is of such a small magnitude that there is little to no negative impact on the tip strength/resistance of the syringe barrel 10 by glass-on-metal contact at the second contact point 82.

As finally shown in FIG. 7, at the third contact point 84 between a side surface of the cylindrical main body 12 of the syringe barrel 10 and the top end 66a of the cylindrical sidewall 66 of the barrel holder 52, a larger force is applied to the side of the cylindrical main body 12. This force applied to the side surface of the cylindrical main body 12 at the third contact point 84 is angled relative to the longitudinal axis (e.g., at 45 degrees), and is at a location on the syringe barrel 10 where the glass-on-metal contact does not affect the structural integrity of the syringe barrel 10. That this, the force applied to the side surface of the cylindrical main body 12 at the third contact point 84 provides/imparts no negative impact on the tip strength/resistance of the syringe barrel 10.

According to aspects of the disclosure, the design of the syringe holding puck 40 as shown in FIGS. 4-7, provides for retention of a syringe barrel 10 therein for performing of an annealing of a silicone coating on the syringe barrel 10 without negatively impacting the tip strength/resistance of the syringe barrel 10, at least in comparison to the prior art holding puck 100 of FIGS. 1A and 1B. Based on structural testing of syringe barrels 10 that were retained by the holding puck 40 and the holding puck 100, it was observed that the tip strength/resistance of the syringe barrel 10 retained by holding puck 40 was at least 53 N greater than the tip strength/resistance of the syringe barrel 10 retained by holding puck 100. Additionally, in no instances was the tip strength/resistance of the syringe barrel 10 retained by holding puck 40 below a level of 40 N, such that the tip strength/resistance of the syringe barrel 10 was determined to be sufficient to reduce the potential for tip breakage.

Beneficially, embodiments of the invention thus provide a syringe barrel holding puck configured to retain and support a syringe barrel therein for performing of an oven-baking process that provides a baked silicone coating on the syringe barrel. The holding puck retains the syringe barrel in a manner that eliminates glass-on-metal contact between the syringe barrel and the holding puck at critical areas of the syringe barrel, such as at tip grooves or shoulders between the tip and the main cylindrical body of the barrel. The holding puck includes a support platform that supports a large part of the weight of the syringe barrel, with additional contact points being provided between the syringe barrel and the holding puck at locations other than the tip grooves.

Although the present disclosure has been described in detail for the purpose of illustration based on what is currently considered to be the most practical and preferred embodiments or aspects, it is to be understood that such detail is solely for that purpose.

## Claims

1. A syringe barrel and a holding puck wherein:
the syringe barrel (10) has a cylindrical main body (12) and a tip (24) positioned at a distal end of the cylindrical main body (12); and
the holding puck (40) is configured for retaining the syringe barrel (10),
wherein the holding puck (40) comprises:
a base member (50);
a barrel holder (52) mounted on the base member (50) and defining a receptacle (54) for receiving at least part of the syringe barrel (10),
**characterized in that** the barrel holder (52) includes:
a cylindrical sidewall (66) having a top end (66a) and a bottom end (66b) and, with the cylindrical sidewall (66) oriented along a longitudinal axis (A_{L}) extending between the top end (66a) and a bottom end (66b), and with the top end (66a) defining a top opening (70); and
a bottom wall (68) positioned at the bottom end (66b) of the cylindrical sidewall (66), the bottom wall (68) including a bottom opening (74) formed therein, with the bottom opening (74) having a diameter that is less than a diameter of the top opening (70) and **in that**
the holding puck (40) further comprises a support bracket (56) affixed to the base member (50), the support bracket (56) including a support platform (78) positioned beneath the barrel holder (52) and oriented orthogonal to the longitudinal axis, the support platform (78) aligned with the bottom opening (74);
wherein, with the syringe barrel (10) being retained in the holding puck (40), a multi-point contact is made between the syringe barrel (10) and the holding puck (40), with the multi-point contact including:
a first contact point (80) between a distal end of the tip (24) and the support platform (78); and
a second contact point (82) between a side surface of the tip (24) and an inner edge of the bottom wall (68) that defines the bottom opening (74).

2. The syringe barrel and the holding puck of claim 1, wherein the multi-point contact further includes a third contact point (84) between a side surface of the cylindrical main body (12) of the syringe barrel (10) and the top end (66a) of the cylindrical sidewall (66) of the barrel holder (52).

3. The syringe barrel and the holding puck of claim 1 or claim 2, wherein the base member (50) comprises a base plate (58) oriented parallel to the longitudinal axis, the base plate (58) including a first extension member (60) and a second extension member (62) spaced apart to define a U-shaped opening (64).

4. The syringe barrel and the holding puck of claim 3, wherein the cylindrical sidewall (66) includes a pair of notches (72) formed on an outer surface thereof, on opposing sides of the cylindrical sidewall (66), and wherein the first extension member (60) and the second extension member (62) interfit with the pair of notches (72) to secure the barrel holder (52) to the base plate (58).

5. The syringe barrel and the holding puck of claim 3 or claim 4, wherein the support platform (78) is positioned within the U-shaped opening (64) defined by the first extension member (60) and the second extension member (62).

6. The syringe barrel and the holding puck of any of claims 3-5, wherein the support bracket (56) comprises a connecting arm (76) extending downward from the support platform (78), with the connecting arm (76) secured to the base plate (58).

7. The syringe barrel and the holding puck of any of claims 1-6, wherein the bottom wall (68) of the barrel holder (52) is sloped downwardly and inwardly from the cylindrical sidewall (66) to the bottom opening (74).

8. The syringe barrel and the holding puck of any of claims 1-7, wherein a diameter of the bottom opening (74) is greater than a diameter of the tip (24) and less than a diameter of the cylindrical main body (12) of the syringe barrel (10).

9. The syringe barrel and the holding puck of any of claims 1-8, wherein at the first contact point (80) between the distal end of the tip (24) and the support platform 78), the support platform (78) supports a majority of a weight of the syringe barrel (10).

10. The syringe barrel and the holding puck of any of claims 1-9, wherein the support bracket (56) is formed of stainless steel.

11. The syringe barrel and the holding puck of any of claims 1-10, wherein the tip (24) of the syringe barrel (10) comprises a luer connection or luer fitting.

12. A system (30) for performing a silicone lubricant annealing of a plurality of syringe barrels (10), the system (30) comprising:
a baking oven (32); and
a retention and transport system (34) for providing the plurality of syringe barrels (10) to the baking oven (32) for annealing of a silicone lubricant applied thereto, the retention and transport system (34) comprising:
a conveyor (42); and
an array of syringe barrels and holding pucks according to claim 1, wherein the holding pucks (40) are affixed to the conveyor (42), with the conveyor (42) configured to translate the array of syringe barrels (10) and holding pucks (40) into the baking oven (32).

13. The system (30) of claim 12, wherein the base member (50) comprises a singular base plate (58) extending along a length of a row of holding pucks (40) of the array of holding pucks (40), with each of the barrel holders (52) in the row of holding pucks (40) secured to the singular base plate (58).

14. The system (30) of claim 13, wherein the support bracket (56) comprises a singular support bracket (56) extending along the length of the row of holding pucks (40), with the singular support bracket (56) affixed to the singular base plate (58), and wherein the singular support bracket (56) includes support platforms (78) thereon corresponding to each of the holding pucks (40) in the row of holding pucks (40).

15. The system (30) of claim 13 or claim 14, wherein the conveyor (42) comprises a belt (46) and a plurality of mounting brackets (48) arranged along a length of the belt (46), and wherein a respective pair of mounting brackets (48) of the plurality of mounting brackets (48) are attached on opposing sides of the belt (46) and are affixed to the singular base plate (58), so as to couple the respective row of holding pucks (40) to the conveyor (42) and provide for advancement thereof.

## Patentansprüche

1. Spritzenzylinder und Haltepuck, wobei:
der Spritzenzylinder (10) einen zylindrischen Hauptkörper (12) und eine Spitze (24) aufweist, die an einem distalen Ende des zylindrischen Hauptkörpers (12) positioniert ist; und
der Haltepuck (40) dazu konfiguriert ist, den Spritzenzylinder (10) zu halten,
wobei der Haltepuck (40) umfasst:
ein Basiselement (50);
ein Zylinderhalter (52), der an dem Basiselement (50) montiert ist und eine Aufnahme (54) definiert, um zumindest einen Teil des Spritzenzylinders (10) aufzunehmen,
**dadurch gekennzeichnet, dass** der Zylinderhalter (52) beinhaltet:
eine zylindrische Seitenwand (66) mit einem oberen Ende (66a) und einem unteren Ende (66b), und wobei die zylindrische Seitenwand (66) entlang einer Längsachse (A_{L}) ausgerichtet ist, die sich zwischen dem oberen Ende (66a) und dem unteren Ende (66b) erstreckt, und wobei das obere Ende (66a) eine obere Öffnung (70) definiert; und
eine Bodenwand (68), die an dem unteren Ende (66b) der zylindrischen Seitenwand (66) angeordnet ist, wobei die Bodenwand (68) eine darin gebildete Bodenöffnung (74) beinhaltet, wobei die Bodenöffnung (74) einen Durchmesser hat, der kleiner ist als der Durchmesser der oberen Öffnung (70) und, dass
der Haltepuck (40) weiter eine an dem Basiselement (50) befestigte Stützhalterung (56) umfasst, wobei die Stützhalterung (56) eine unter dem Zylinderhalter (52) angeordnete und orthogonal zu der Längsachse ausgerichtete Stützplattform (78) beinhaltet, wobei die Stützplattform (78) mit der unteren Öffnung (74) ausgerichtet ist;
wobei, indem der Spritzenzylinder (10) in dem Haltepuck (40) gehalten wird, ein Mehrpunktkontakt zwischen dem Spritzenzylinder (10) und dem Haltepuck (40) hergestellt wird, wobei der Mehrpunktkontakt beinhaltet:
einen ersten Kontaktpunkt (80) zwischen einem distalen Ende der Spitze (24) und der Stützplattform (78); und
einen zweiten Kontaktpunkt (82) zwischen einer Seitenfläche der Spitze (24) und einer inneren Kante der Bodenwand (68), welche die Bodenöffnung (74) definiert.

2. Spritzenzylinder und Haltepuck nach Anspruch 1, wobei der Mehrpunktkontakt weiter einen dritten Kontaktpunkt (84) zwischen einer Seitenfläche des zylindrischen Hauptkörpers (12) des Spritzenzylinders (10) und dem oberen Ende (66a) der zylindrischen Seitenwand (66) des Zylinderhalters (52) beinhaltet.

3. Spritzenzylinder und Haltepuck nach Anspruch 1 oder Anspruch 2, wobei das Basiselement (50) eine Basisplatte (58) umfasst, die parallel zu der Längsachse ausgerichtet ist, wobei die Basisplatte (58) ein erstes Verlängerungselement (60) und ein zweites Verlängerungselement (62) beinhaltet, die voneinander beabstandet sind, um eine U-förmige Öffnung (64) zu definieren.

4. Spritzenzylinder und Haltepuck nach Anspruch 3, wobei die zylindrische Seitenwand (66) ein Paar Kerben (72) beinhaltet, die an einer Außenseite davon auf gegenüberliegenden Seiten der zylindrischen Seitenwand (66) gebildet sind, und wobei das erste Verlängerungselement (60) und das zweite Verlängerungselement (62) mit dem Paar Kerben (72) ineinandergreifen, um den Zylinderhalter (52) an der Basisplatte (58) zu befestigen.

5. Spritzenzylinder und Haltepuck nach Anspruch 3 oder Anspruch 4, wobei die Stützplattform (78) innerhalb der U-förmigen Öffnung (64) positioniert ist, die durch das erste Verlängerungselement (60) und das zweite Verlängerungselement (62) definiert ist.

6. Spritzenzylinder und Haltepuck nach einem der Ansprüche 3-5, wobei die Stützhalterung (56) einen Verbindungsarm (76) umfasst, der sich von der Stützplattform (78) nach unten erstreckt, wobei der Verbindungsarm (76) an der Basisplatte (58) befestigt ist.

7. Spritzenzylinder und Haltepuck nach einem der Ansprüche 1-6, wobei die Bodenwand (68) des Zylinderhalters (52) von der zylindrischen Seitenwand (66) zu der unteren Öffnung (74) hin abwärts und nach innen geneigt ist.

8. Spritzenzylinder und Haltepuck nach einem der Ansprüche 1-7, wobei ein Durchmesser der unteren Öffnung (74) größer als ein Durchmesser der Spitze (24) und kleiner als ein Durchmesser des zylindrischen Hauptkörpers (12) des Spritzenzylinders (10) ist.

9. Spritzenzylinder und Haltepuck nach einem der Ansprüche 1-8, wobei an dem ersten Kontaktpunkt (80) zwischen dem distalen Ende der Spitze (24) und der Stützplattform (78) die Stützplattform (78) einen Großteil des Gewichts des Spritzenzylinders (10) trägt.

10. Spritzenzylinder und Haltepuck nach einem der Ansprüche 1-9, wobei die Stützhalterung (56) aus Edelstahl gebildet ist.

11. Spritzenzylinder und Haltepuck nach einem der Ansprüche 1-10, wobei die Spitze (24) des Spritzenzylinders (10) eine Luer-Verbindung oder einen Luer-Anschluss umfasst.

12. System (30) zum Durchführen von Tempern eines SilikonSchmiermittels von mehreren Spritzenzylindern (10), wobei das System (30) umfasst:
einen Brennofen (32); und
ein Rückhalte- und Transportsystem (34) zum Bereitstellen der Vielzahl von Spritzenzylindern (10) für den Brennofen (32) zum Tempern eines darauf aufgetragenen Silikonschmiermittels, wobei das Rückhalte- und Transportsystem (34) umfasst:
ein Förderband (42); und
eine Anordnung von Spritzenzylindern und Haltepucks nach Anspruch 1, wobei die Haltepucks (40) an dem Förderband (42) befestigt sind, wobei das Förderband (42) dazu konfiguriert ist, die Anordnung von Spritzenzylindern (10) und Haltepucks (40) in den Brennofen (32) zu transportieren.

13. System (30) nach Anspruch 12, wobei das Basiselement (50) eine einzelne Basisplatte (58) umfasst, die sich entlang einer Länge einer Reihe von Haltepucks (40) der Anordnung von Haltepucks (40) erstreckt, wobei jeder der Zylinderhalter (52) in der Reihe von Haltepucks (40) an der einzelnen Basisplatte (58) befestigt ist.

14. System (30) nach Anspruch 13, wobei die Stützhalterung (56) eine einzelne Stützhalterung (56) umfasst, die sich entlang der Länge der Reihe von Haltepucks (40) erstreckt, wobei die einzelne Stützhalterung (56) an der einzelnen Basisplatte (58) befestigt ist, und wobei die einzelne Stützhalterung (56) darauf Stützplattformen (78) aufweist, die jeweils den Haltepucks (40) in der Reihe von Haltepucks (40) entsprechen.

15. System (30) nach Anspruch 13 oder Anspruch 14, wobei das Förderband (42) einen Riemen (46) und eine Vielzahl von Montagehalterungen (48) umfasst, die entlang der Länge des Riemens (46) angeordnet sind, und wobei jeweils ein Paar von Montagehalterungen (48) der Vielzahl von Montagehalterungen (48) an gegenüberliegenden Seiten des Riemens (46) befestigt und an der einzelnen Basisplatte (58) angebracht sind, um die jeweilige Reihe von Haltepucks (40) mit dem Förderband (42) zu koppeln und deren Vorschub zu ermöglichen.

## Revendications

1. Cylindre de seringue et coupelle de maintien, dans lesquels :
le cylindre de seringue (10) a un corps principal cylindrique (12) et une pointe (24) positionnée au niveau d'une extrémité distale du corps principal cylindrique (12) ; et
la coupelle de maintien (40) est configurée pour retenir le cylindre de seringue (10),
dans lesquels la coupelle de maintien (40) comprend :
un organe de base (50) ;
un porte-cylindre (52) monté sur l'organe de base (50) et définissant un réceptacle (54) pour recevoir au moins une partie du cylindre de seringue (10),
**caractérisés en ce que** le porte-cylindre (52) comprend :
une paroi latérale cylindrique (66) ayant une extrémité supérieure (66a) et une extrémité inférieure (66b) et, la paroi latérale cylindrique (66) étant orientée le long d'un axe longitudinal (A_{L}) s'étendant entre l'extrémité supérieure (66a) et une extrémité inférieure (66b) et, l'extrémité supérieure (66a) définissant une ouverture supérieure (70) ; et
une paroi inférieure (68) positionnée au niveau de l'extrémité inférieure (66b) de la paroi latérale cylindrique (66), la paroi inférieure (68) comprenant une ouverture inférieure (74) formée dedans, l'ouverture inférieure (74) ayant un diamètre qui est inférieur au diamètre de l'ouverture supérieure (70),
et **en ce que** la coupelle de maintien (40) comprend en outre un support de soutien (56) fixé à l'organe de base (50), le support de soutien (56) comprenant une plateforme de soutien (78) positionnée sous le porte-cylindre (52) et orientée orthogonalement à l'axe longitudinal, la plateforme de soutien (78) étant alignée avec l'ouverture inférieure (74) ;
dans lesquels, avec le cylindre de seringue (10) retenu dans la coupelle de maintien (40), un contact multipoint est établi entre le cylindre de seringue (10) et la coupelle de maintien (40), le contact multipoint comprenant :
un premier point de contact (80) entre une extrémité distale de la pointe (24) et la plateforme de soutien (78) ; et
un deuxième point de contact (82) entre une surface latérale de la pointe (24) et un bord interne de la paroi inférieure (68) qui définit l'ouverture inférieure (74).

2. Cylindre de seringue et coupelle de maintien selon la revendication 1, dans lesquels le contact multipoint comprend en outre un troisième point de contact (84) entre une surface latérale du corps principal cylindrique (12) du cylindre de seringue (10) et l'extrémité supérieure (66a) de la paroi latérale cylindrique (66) du porte-cylindre (52).

3. Cylindre de seringue et coupelle de maintien selon la revendication 1 ou 2, dans lesquels l'organe de base (50) comprend une plaque de base (58) orientée parallèlement à l'axe longitudinal, la plaque de base (58) comprenant un premier organe d'extension (60) et un second organe d'extension (62) espacés de manière à définir une ouverture en forme de U (64).

4. Cylindre de seringue et coupelle de maintien selon la revendication 3, dans lesquels la paroi latérale cylindrique (66) comprend une paire d'encoches (72) formées sur sa surface externe, sur des côtés opposés de la paroi latérale cylindrique (66), et dans lesquels le premier organe d'extension (60) et le second organe d'extension (62) s'emboîtent avec la paire d'encoches (72) afin de fixer le porte-cylindre (52) à la plaque de base (58).

5. Cylindre de seringue et coupelle de maintien selon la revendication 3 ou 4, dans lesquels la plateforme de soutien (78) est positionnée à l'intérieur de l'ouverture en forme de U (64) définie par le premier organe d'extension (60) et le second organe d'extension (62).

6. Cylindre de seringue et coupelle de maintien selon l'une des revendications 3 à 5, dans lesquels le support de soutien (56) comprend un bras de liaison (76) s'étendant vers le bas depuis la plateforme de soutien (78), le bras de liaison (76) étant fixé à la plaque de base (58).

7. Cylindre de seringue et coupelle de maintien selon l'une des revendications 1 à 6, dans lesquels la paroi inférieure (68) du porte-cylindre (52) est inclinée vers le bas et vers l'intérieur depuis la paroi latérale cylindrique (66) jusqu'à l'ouverture inférieure (74).

8. Cylindre de seringue et coupelle de maintien selon l'une des revendications 1 à 7, dans lesquels un diamètre de l'ouverture inférieure (74) est supérieur à un diamètre de la pointe (24) et inférieur à un diamètre du corps principal cylindrique (12) du cylindre de seringue (10).

9. Cylindre de seringue et coupelle de maintien selon l'une des revendications 1 à 8, dans lesquels, au niveau du premier point de contact (80) entre l'extrémité distale de la pointe (24) et la plateforme de soutien (78), la plateforme de soutien (78) supporte la majeure partie du poids du cylindre de seringue (10).

10. Cylindre de seringue et coupelle de maintien selon l'une des revendications 1 à 9, dans lesquels le support de soutien (56) est formé d'acier inoxydable.

11. Cylindre de seringue et coupelle de maintien selon l'une des revendications 1 à 10, dans lesquels la pointe (24) du cylindre de seringue (10) comprend une connexion Luer ou un raccord Luer.

12. Système (30) pour réaliser un recuit de lubrifiant silicone d'une pluralité de cylindres de seringue (10), le système (30) comprenant :
un four de cuisson (32) ; et
un système de maintien et de transport (34) pour fournir la pluralité de cylindres de seringue (10) au four de cuisson (32) pour le recuit d'un lubrifiant silicone appliqué à ceux-ci, le système de maintien et de transport (34) comprenant :
un transporteur (42) ; et
un réseau de cylindres de seringue et de coupelles de maintien selon la revendication 1, où les coupelles de maintien (40) sont fixées au transporteur (42), le transporteur (42) étant configuré pour effectuer une translation du réseau de cylindres de seringue (10) et de coupelles de maintien (40) dans le four de cuisson (32).

13. Système (30) selon la revendication 12, dans lequel l'organe de base (50) comprend une plaque de base unique (58) s'étendant sur une longueur d'une rangée de coupelles de maintien (40) du réseau de coupelles de maintien (40), chacun des porte-cylindres (52) dans la rangée de coupelles de maintien (40) étant fixé à la plaque de base unique (58).

14. Système (30) selon la revendication 13, dans lequel le support de soutien (56) comprend un support de soutien unique (56) s'étendant sur la longueur de la rangée de coupelles de maintien (40), le support de soutien unique (56) étant fixé à la plaque de base unique (58), et dans lequel le support de soutien unique (56) comprend des plateformes de soutien (78) sur celui-ci correspondant à chacune des coupelles de maintien (40) dans la rangée de coupelles de maintien (40).

15. Système (30) selon la revendication 13 ou 14, dans lequel le transporteur (42) comprend une courroie (46) et une pluralité de supports de montage (48) agencés sur une longueur de la courroie (46), et dans lequel une paire respective de supports de montage (48) de la pluralité de supports de montage (48) est fixée sur des côtés opposés de la courroie (46) et fixée à la plaque de base unique (58), de manière à coupler la rangée respective de coupelles de maintien (40) au transporteur (42) et à permettre son avancement.
